# EUROPEAN PATENT SPECIFICATION

(11) **EP 0 491 938 B1**
(45) Date of publication and mention of the grant of the patent: **09.09.1998**
(21) Application number: 91914442.8
(22) Date of filing: 28.06.1991
(51) Int. Cl.: A01H 1/02, A01H 5/10

(54) **CORN OIL**
MAISÖL
HUILE DE MAIS

(30) Priority: 19.07.1990 US 554526; 23.07.1990 US 556071
(43) Date of publication of application: 01.07.1992
(73) Proprietor: AGRIGENETICS, INC., San Diego, California (US)
(72) Inventor: ALEXANDER, William, Larry, Breckenridge, MN 56520 (US); JELLUM, Milton, Delbert, Griffin, GA 30223 (US)
(74) Representative: Crisp, David Norman
(86) International application number: US9104626
(87) International publication number: WO9201367

(56) References cited:
- EP-A- 0 323 753
- WO-A-90/00856
- FR-A- 2 617 675
- US-A- 4 627 192
- Journal Agricultural Food Chem., volume 18, no. 3, 1970, M.D. Jellum: "Plant introductions of maize as a source of oil with unusual fatty acid composition", pages 365-370, see page 368, right-hand column, line 1 - page 369, right-hand column, line 5; tale IV (cited in the application)
- CAB Abstracts 0516471 OPO46-11070, E.J. Weber et al.: "Breeding for lipid composition in corn", & Journal of the American Oil Chemists' Society, 1975, 52(9): 370-373, see the wole article
- Kirk-Othmer: "Encyclopedia of chemical technology", 1980, John Wiley & Sons, (New York, US), volume 9, chapter "Fats and Fatty oils", pages 795-831, see tables 1-3
- Crop Science, volume 24, 1984, N.W. Widstrom et al.: "Chromosomal location of genes controlling oleic and linoleic acid composition in the germ oil of two maize inbreds", pages 1113-1115, see the whole document (cited in the application)
- R.W. Allard: "Principles of plantbreeding", 1960, John Wiley & Sons, (New York, US) Chapter 16 "Responses to selection and the genetic oganisation of populations", pages 182-196; chapter 21 "Selection in cross- pollinated crops", pages 252-262; chapter 23, "Recurrent selection", pages 282-302, see page 184, line 1 - page 189, line 10; figures 16.1-16.4; page 258, line 19 - page 260, line 23
- N.W. Simmonds: "Principles of crop improvement", 1979, Longman (London, GB) Chapter 5 "Breeding plans", paragraphs 5.4 and 5.5, pages 135- 161, see the whole document

## Description

The present invention relates to a corn oil. In particular, the invention relates to the production of corn oil generally characterized by reproducibly high levels of oleic acid, as measured by gas-liquid chromatography (GLC).

Corn oil is composed of saturated and unsaturated fatty acids with carbon chain lengths ranging from 12 to 24. Approximately 95% or more of the total oil content is composed of palmitic (16:0), stearic (18:0), oleic (18:1), and linoleic (18:2) acids, Jellum, J. Agric. Food Chem., 18:365-70 (1970).

During the past 25 years, inbred lines and hybrids of corn (Zea mays L.) have been documented that display oleic acid compositions varying over a broad range, see, for example, Weber & Alexander, J.Am.Oil Chem.Soc. 51:512A (1974). Jellum, J. Heredity 57:243-44 (1966), has documented corn oleic acid values that varied between 22.9% and 45.5% among inbred lines and the progeny of reciprocal crosses thereof. Subsequent testing of a large number of corn introductions from around the world revealed oleic acid content for single kernels ranging from 13.7% to 64.3%. Jellum, J. Agric. Food Chem., supra.

More recently, six corn inbred lines characterized by different fatty acid contents were used in testing of mean parental, F1, F2, and backcross levels of oleic acid, and a range of 15.1% to 50.3% was documented. Widstrom & Jellum, Crop Sci. 15:44-46 (1975). Generally, F1 and F2 mean values were intermediate to either parental values.

A wide range of oleic acid content has thus been documented for corn, but no value greater than 64.3% has been found. This 64.3% value was obtained for one seed from a single S1 ear from a variable Chilean corn population, see Jellum, J. Agric. Food Chem., supra ("PI No. 303851" in Table III). In Table IV, Jellum also shows an Australian inbred line, designated "CI-7B," which has an oleic acid content of 63.5%. Id. Inquiry in Australia has indicated, however, that germplasm of CI-7B is no longer available to the public.

As to the heritability for oleic acid content in corn, no consistent pattern has been documented in inbreds and in their reciprocal crosses by Jellum, J. Heredity, supra, who attributed the observed phenomenon to a "diversity of gene action for certain fatty acids," including oleic acid. Although reports have appeared from time to time favoring a "single gene" hypothesis for the control of corn oleic acid, see Widstrom & Jellum, supra, and Poneleit & Alexander, Science 147:1585-86 (1965), more recent findings have indicated "a mode of inheritance ... more complicated [than the single-gene hypothesis] ... due primarily to additive gene effects," Weber, Biochem. Genet. 21:1-10 (1983). In de la Roche et al., Crop Sci. 11:856-59 (1971), it is suggested that oleic acid content in corn is under the control of genes at loci in addition to the ln locus, where an oleic content-influencing gene is found. More recently, genes affecting oleic acid content have been localized, in various corn lines, to one or more of chromosomes 1, 4 and 5; Widstrom & Jellum, Crop Sci. 24:1113-15 (1984); Shadley & Weber, Can. J. Genet. Cytol. 22:11-19 (1980).

Accordingly, the published literature on oleic acid content in corn indicates the presence of diverse genes, located on different chromosomes, that affect oleic acid content in a manner not clearly understood. This fact, combined with the virtual absence of information regarding the molecular biology of fatty acid profile in corn, has complicated the task of modifying the oleic acid level in corn and, in particular, has rendered the breeding endeavour of selecting for corn oleic acid content highly unpredictable a priori. Moreover, there has been no basis to date for a reasonable expectation of success in obtaining mean oleic acid levels in excess of 45% for commercially acceptable corn genotypes.

Percentages and ratios given herein are by weight, and temperatures are in degress Celsius unless otherwise indicated. Where necessary to better exemplify the invention, percentages and ratios may be cross-combined.

In general, the present invention relates to a corn oil having a percentage of oleic acid that is comparable to that of high oleic canola oil and high oleic sunflower oil.

More specifically, the present invention provides a corn oil having a mean oleic acid content of at least 75 % relative to the total oil which has been extracted from a substantially homogeneous assemblage of corn seeds having a mean oleic acid content of at least 75 % by weight relative to the total fatty acid content of said seeds.

Desirably, the oil is obtained from seed which has an oleic acid content of between about 75 % and 78 %, between about 78 % and 82 % or greater than 82 % by weight relative to the total fatty acid content of the seed (hereinafter expressed as percent by weight or simply percent). In the most preferred embodiment, the oil is obtained from seed which has an oleic acid content of greater than 80 %. Desirably, the oil is obtained from seeds having a mean oleic acid content between about 75 % and 80 % by weight. The seeds have a mean oleic acid content of at least 75 %, or even greater than about 80 % by weight. The invention also relates to an oil obtained from a commercially acceptable corn variety having a mean oleic acid content of kernel bulks of at least 75 % by weight. Conveniently, the kernel bulks have a mean oleic acid content between about 75 % and 80 % by weight. The kernel bulks have a mean oleic acid content of at least 75 % or desirably at least 80 % by weight.

Other features and advantages of the present invention will become apparent from the following detailed description. It should be understood, however, that the detailed description and the specific examples, while indicating preferred embodiments of the invention, are given by way of illustration only, since various changes and modifications within the scope of the invention will become apparent to those skilled in the art from this detailed description.

### DEPOSIT INFORMATION

Inbred seeds of Zea mays L. strain F₄ of LH85 x 85-205-6 have been placed on deposit with In Vitro International, Inc., Linthicum, Maryland 21090 on July 19, 1990, and have been assigned IVI No. 10254.

### DETAILED DESCRIPTION OF THE PREFERRED EMBODIMENTS

In order to provide an understanding of a number of terms used in the specification and claims herein, the following definitions are provided.

"Selection" - Occurs when plants with desired phenotypes or genotypes are chosen for additional plant breeding procedures and breeding projects.

"Intermating" - Is used synonymously with "sibbing" to denote the practice of planting seeds of selected plant phenotypes in individual rows, such that normal germination, emergence and plant maturation occur, and (at the onset of pollen-shed and silk extrusion) systematically crossing plants from each of these rows to plants from as many other rows as possible, thereby to maximize the number of crosses between unrelated individuals in the population.

"Variety" - Refers to a group of plants within a species, such as Zea mays L., which share certain constant characters that separate them from other possible varieties within that species. While possessing at least one distinctive trait, a variety can also be characterized by a substantial amount of variation between individuals within the variety, based primarily on the Mendelian segregation of traits among the progeny of succeeding generations.

"Line" - A line as distinguished from "variety" and "cultivar" refers to a group of plants which are substantially uniform in their traits except that there is relatively minor variation within the group and such variation can be characterized. The decreased variation within this group has generally (although not exclusively) resulted from several generations of self-pollination (selfing).

"True Breeding" - A line is considered "true breeding" for a particular trait if it is genetically homozygous for that trait to the extent that when the variety is self-pollinated, no significant amount of independent segregation of the trait among progeny is observed.

"Significant Variation" - In this regard, significant variation can be adjudged, for example, relative to the variation in oleic value that is attributable to kernel position on an ear, i.e., a variation of between about 4% and 5%. See Jellum, Crop Sci. 7:593-95 (1967). Thus, high oleic corn material of the present invention is that characterized by a variation in single-kernel oleic values of about 5% or less is deemed to be true-breeding for the high oleic trait. As an alternative definition of true breeding for oleic acid content, the variety when self-pollinated will provide progeny wherein 95% or more of the seeds of the progeny have an oleic acid content above the specified minimum. Thus, an oleic acid content of "at least 75%" will for a true-breeding self-pollinated variety have more than about 95% of the progeny seeds of an oleic acid content of at least 75%. As another example, a corn variety according to the present invention is true-breeding for an oleic acid content of 75% (as opposed to "at least 75%") if, when the variety is self-pollinated, about 95% or more of the progeny corn seeds have an oleic acid content of about 75%, i.e., in about 95% or more of the progeny seeds, there is no significant variation from an oleic acid content of 75%. The relative importance of certain environmental factors (such as planting date, ear position, location and year) and of genetic factors in determining the fatty acid composition of corn oil has been reported by Jellum, Crop Sci. (1967), supra. The results (Table 2) of combined analysis showed that the oil of kernels from the tip of the ear contained less oleic acid and more linoleic acid than did oil from kernels of other positions of the ear. For the inbreds tested in Table 2, the oleic acid content varied by 0.6% to 4.8% as a result of kernel position. The highest oleic acid values generally were obtained from kernels located in the middle or base of the ear, and the lowest oleic acid values resulted from kernels located at the ear tip.

"Oleic Acid Content" and "Oleic Value" - These terms are used synonymously and interchangeably with relation to measurements of the proportion of oleic acid to total fatty acids present in corn oil which is extracted from single seeds (using the whole- or half-seed technique, as described below) or from bulked seed. Since the oleic values set out in this description are generally obtained from GLC analyses, the reported proportions of oleic acid are essentially by weight. When oleic acid content or oleic value are expressed as a percent or a percent by weight, it is to be understood that such percentage is relative to the total fatty acid content of the seed(s).

"Bulked Seed" - can be constituted, for example, from a plurality of seeds of a single cob (a "kernel bulk"), from the combined seed from all or a particular part of a genetically related family of plants, or from the seed of a plant introduction (defined below).

"Corn Oil" in the present context is a vegetable oil produced via a process comprising the extraction of oil from corn seed, preferably bulked seed constituted from seeds containing oil with a mean oleic acid content in the same general range, say, greater than 50%. In a preferred embodiment, corn oil of the present invention is obtained by a process entailing extraction of oil from a collection of seeds that differ in oleic acid content over a range of no more than about 5% by weight; such a collection is said to be a "substantially homogeneous assemblage" of seeds (defined below).

A "Commercially Acceptable Genotype" is used to refer to any genotype including genotypes of inbreds and hybrids which yield plants having agronomically acceptable traits (such as yield, moisture, rate of dry-down, stalk lodging, vigor, root lodging, disease resistance, insect resistance and the like) such that (A) with respect to inbreds, these traits allow the economical use of this genotype in the production of hybrid seed, or (B) with respect to hybrids, the acceptable traits are present in the hybrid plants when grown in a farmer's field.

A "Plant Introduction" (P.I.) - is a sample of seeds of the same species (e.g., Zea mays L.) that can be grown into plants having a common discernible (gross) morphology. Generally designated by country of origin, a P.I. often represents germplasm native and/or adapted to that country, and hence may embody considerable genetic variability. A P.I. can also represent the germplasm of an inbred line.

"Homogeneous Assemblage" - The term is intended to refer to a group of seeds which are homogeneous (do not have significant variation) for a given characteristic. One example of a group of seeds would be 2,000 randomly-selected seeds from one acre of corn.

"Mean Oleic Acid Content" - Is the average of oleic acid values obtained for a designated genotype or family of genotypes. The oleic acid values are determined from bulked seed (defined above) of the genotype or from nine or more kernels equally representing the top, middle and base portions of the ear.

A general source for germplasm has been identified, in the form of a specific subgroup of corn varieties and lines comprised, respectively, of plants producing individual seeds from which high oleic material can be generated predictably in accordance with the description herein. In particular, it has been discovered that corn varieties and lines can be produced using starting material chosen from the aforementioned subgroup and subjected to intermating and selection of the top 1-20% of oleic acid-producing plants, followed by cyclic self-pollination and selection of genotypes (lines) which have a mean oleic acid content of at least 75% by weight. Moreover, the high oleic characters developed thus can be readily irtrogressed (transferred) into other genetic backgrounds, for example, by backcrossing a high oleic inbred line of the present invention with a recurrent parent having one or more desired agronomic characteristics other than high oleic value. The product of backcrossing to an elite recurrent parent, followed by four to eight selfing generations, is an inbred corn line which is agronomically desirable in addition to having a mean oleic acid percentage of at least 45%.

A high oleic oil produced in this manner would be expected to possess a sterol constituency typifying corn oils per se, see Itoh et al., J.Am.Oil Chem.Soc. 50:122-25 (1973), particularly with regard to the 24-methyl-Δ²³ sterols described by Itoh et al., Phyto-chem. 20:1353-56 (1981), at least one of which (24-methyl-23-dihydrolophenol) is thought to be unique to corn oil. The high oleic oil of the present invention could be used as a raw material in making other oil products which, although sufficiently modified so as not to be recognizable as "corn oil," would retain a high oleic acid content. Identification of individual oils in mixtures was achieved in an article by Abu-Hadeed, J.Am.Oil Chem.Soc. 65:1922 (1988). Table 1 of this reference shows the differences for specific components between economically important vegetable oils. Table 2 shows the threshold value of the characteristic component of corn oil to be 2.20 mg/g of β-Sitosterol.

Starting materials are known and readily available from the USDA Plant Introduction Station at Iowa State University (Ames, Iowa). P.I.'s from the Ames collection, which embody varieties or lines that are exemplary sources of germplasm to use in the present invention, are listed in Table 1 herein. The varieties and lines represented by such accessions after selfing produce bulked seed from individual ears that, while possibly displaying a variable overall oleic acid content, comprise a significant percentage of individual seeds having oleic values that are about 45% or greater, but typically less than about 55%. However, there were wide variations in oleic acid content between individual ears obtained by selfing the indicated lines. For example, PI 303851 (Chile) produced seeds having an oleic acid content of from 32.93% to 48.49%. The oleic acid content of kernels of selected ears is shown in Table 1.

**Table 1**

| EXEMPLARY SOURCES OF GERMPLASM Single Seed Oil Samples of Plant Introductions (S0 ears) (Ames Collection) | | | | |
|---|---|---|---|---|
| P.I. No. | Origin | Oleic Acid (%) | | |
| 162573 | Argentina | 42.04, | 44.58, 52.75, | 40.86, 39.18 |
| 186218 | " | 45.16, | 44.03, 47.27, | 46.29, 45.88 |
| 180163 | Austria | 40.54, | 40.81, 42.74, | 49.04, 43.09 |
| 303851 | Chile | 48.49, | 45.52, 32.93, | 44.77, 40.53 |
| 291391 | China | 49.50, | 43.53, 43.18, | 47.32, 44.78 |
| 303878 | Japan | 43.62, | 47.09, 41.66, | 42.49, 48.32 |
| 303889 | Japan | 39.78, | 44.23, 40.36, | 49.26, 42.72 |
| 181839 | Lebanon | 48.25, | 53.63, 61.15, | 49.49, 57.15 |
| 209135 | Puerto Rico | 51.55, | 32.79, 42.88, | 40.08, 37.38 |
| 228182 | Russia | 50.40, | 46.95, 45.47, | 48.89, 47.02 |
| 236995 | Siberia | 44.58, | 32.53, 30.44, | 42.25, 43.86 |
| 180230 | United Kingdom | 46.60, | 40.17, 44.00, | 33.46, 46.41 |
| 218162 | USA (Arizona) | 36.82, | 35.02, 43.19, | 44.33, 42.53 |
| 213701 | USA (Iowa) | 46.51, | 38.97, 40.46, | 45.35, 38.62 |
| 217406 | USA (Iowa) | 48.72, | 37.14, 42.48, | 35.61, 41.88 |
| 217409 | USA (Iowa) | 50.78, | 37.01, 40.16, | 35.94, 40.15 |
| 218141 | USA (New Mex.) | 46.58, | 44.02, 35.05, | 39.97, 37.92 |
| 218142 | USA (New Mex.) | 42.65, | 46.45, 49.84, | 40.33, 34.53 |
| 213796 | USA (N. Dak.) | 40.27, | 48.19, 41.86, | 41.41, 39.86 |
| 245130 | USA (Rd. Island) | 41.68, | 44.89, 40.54, | 43.53, 40.28 |
| 317680 | USA (S. Dakota) | 43.20, | 44.27, 44.01, | 40.50, 44.89 |

Other sources for publicly-available corn germplasm include the U.S. Department of Agriculture's National Seed Storage Laboratory (Colorado State University, Fort Collins, Colorado) and Centro Internacional de Mejoramiento de Maiz y Trigo (International Center of Corn and Wheat Improvement), Londres 40, Apertado Postal 6-641, 06600 Mexico, D.F., Mexico.

In accordance with the present invention, bulk seed of a suitable startin variety or line is screened to identify those seeds having an oleic acid content of at least about 45%. The screening can be effected, for example, by a half-seed technique, in which the seed scutulum is excised and the oil extracted is assayed by GLC, see Jellum & Worthington, Crop Sci. 6:251-53 (1966), or by similarly analyzing oil extracted from a whole seed. The latter approach does not save the embryo for germination. The screening can be performed before an initial selfing step or, if a greater degree of segregation is desired, after a self-pollination of plants grown from the bulk seed.

The embryos from seeds thus identified as having an oleic value of about 45% or more can then be germinated in the conventional manner, grown out to maturity, and self-pollinated to increase supply of the selected germplasm. At this stage and others of the method described herein, the growing conditions are preferably such that heat and moisture stress are avoided. Seeds from selfed plants are then grown out and crossed with individuals of the same selfed population ("sib pollination") in such a way as to maximize the combinations of pollinations within the population.

The offspring of the sib pollination are again screened for high oleic value, and a percentage of progeny representing an upper range of oleic acid content, preferably the upper 10%, are selected. The upper range employed for selection is chosen to balance the rapidity with which oleic acid content is raised in a population versus the quantitative level of oleic value ultimately attained for that population. Lower selection pressure will maintain a higher level of genetic variability within the population. A further cycle of sib pollination and selection is then carried out, using plants grown from seed representing the selected upper range. Generally, within two to four such cycles of recurrent selection one can proceed from starting material, chosen from the subgroup defined above, to a corn variety that is characterized by mean oleic values for kernel bulks that are 75% or greater.

Between two to four additional cycles of self-pollination and selection will typically be needed to reach a state of relative homogeneity, at least with respect to high oleic value, that is indicative of a true-breeding variety or line. In any event, single-kernel oleic values in excess of 75%, preferably around 85%, can be achieved in association with such inbreeding of high oleic varietal material within the present invention.

The fatty acid composition of corn seeds developed in the breeding program can be determined by GLC in accordance with the procedures described in Jellum and Worthington, supra. GLC analysis can be conducted on a five- (or more) kernel bulk sample and on a one-half kernel sample, which allows the planting of the remaining half-seed for further breeding.

Elite high oleic acid corn lines (oleic acid lines and oleic lines are used interchangeably herein) can be developed by crossing the high oleic line (derived from a high oleic acid P.I. population) with an agronomically elite line for a given maturity region. For example, LH85 is an elite corn inbred line which has an oleic acid content ranging from 26.1% to 35.9%. 85-205-6 is a high oleic acid line developed by the procedures of this invention. By crossing LH85 with 85-205-6, followed with two to four generations of selfing and selection of high oleic lines with agronomically desirable traits, the lines resulting from this breeding effort exhibit high oleic acid content along with acceptable agronomic traits such as plant vigor, good stalks and roots, disease resistance, and the like. Crossing high oleic lines with a number of elite lines and selfing and selecting from these crosses can produce numerous new high oleic acid corn lines. These lines can be adapted to any maturity region desired by selecting the appropriate maturity level in the elite corn parent and selecting for the desired maturity in subsequent selfing generations. The high oleic acid lines developed by the present invention can be used as one or more parents in corn hybrids.

Also, one or more backcrosses to the elite recurrent parent can be accomplished to incorporate a higher percentage of the elite germplasm characteristics while retaining the high oleic trait. For example, the cross PA91 x 85-205-21 was backcrossed to PA91 (designated as PA91²85-205-21), which was then selfed, and selections made for high oleic lines. PA91 is an elite 128-day maturity corn inbred line developed it Pennsylvania State University. 85-205-21 is a high oleic acid line developed by the procedures described herein. The five kernel bulk GLC assay of ear 19 of this backcross had an oleic acid content of 56.4%. The half-kernel GLC assay of kernel 31 from ear 19 had a 72.4% oleic acid level. The remaining half-kernel 31 from ear 19 is planted and used in further breeding procedures, such as (1) further selfing and selection, (2) making additional backcrosses to the PA91 recurrent parent to incorporate more of the PA91 elite background, (3) crossing with other elite lines to incorporate desired traits while maintaining the high oleic acid trait, and (4) conducting other conventional breeding procedures to develop commercially viable corn inbreds and hybrids.

As mentioned above, an inbred line that is true-breeding for a high oleic acid phenotype according to the present invention is advantageously employed in a backcrossing program to introgress the high oleic trait into other, more agronomically desirable lines. For example, a true-breeding inbred line of the present invention can be the donor parent for backcrossing to a waxy corn line, to thereby produce a high oleic wary hybrid or line. In this regard, a "hybrid" would be an offspring obtained by crossing parent plants of different lineage.

As disclosed, for example, by Coe et al., in Corn and Corn Improvement (3d ed.), Sprague, G.F. and Dudley, J.W., Eds., p. 142-143 (American Society of Agronomy, Madison, Wis., 1988) (hereinafter "Coe et al. (1988)"), the waxy type of kernel is so unique and its expression so unconfounded that the waxy trait is conventionally used as a universal marker. The waxy endosperm chips away evenly when cut with a blade, leaving a smooth, opaque surface, while normal endosperm breaks unevenly and leaves an irregular, translucent surface. In addition, the starch in the outer surface of a non-waxy endosperm stains blue, turning quickly to black, with an iodine (I₂)-potassium iodide (KI) solution, while that of material homozygous for the waxy allele (wx1) stains reddish brown, turning soon to dark brown.

The uniqueness of the waxy trait allows for the ready backcrossing to a recurrent waxy parent of progeny that are (high oleic x waxy) hybrids, according to the present invention, against a donor waxy (wx1/wx1) parent. That is, progress can be readily monitored for a backcrossing generation whereby the germplasm contribution of the high oleic donor, save for the expression of a mean oleic value of 45% or greater, is virtually eliminated.

Introgression of a high oleic phenotype as described above can also be accomplished with regard to genetic backgrounds characterized by traits other than waxy. Illustrative of traits that could be combined with a high oleic phenotype, pursuant to the present invention, are those listed in Table 2.

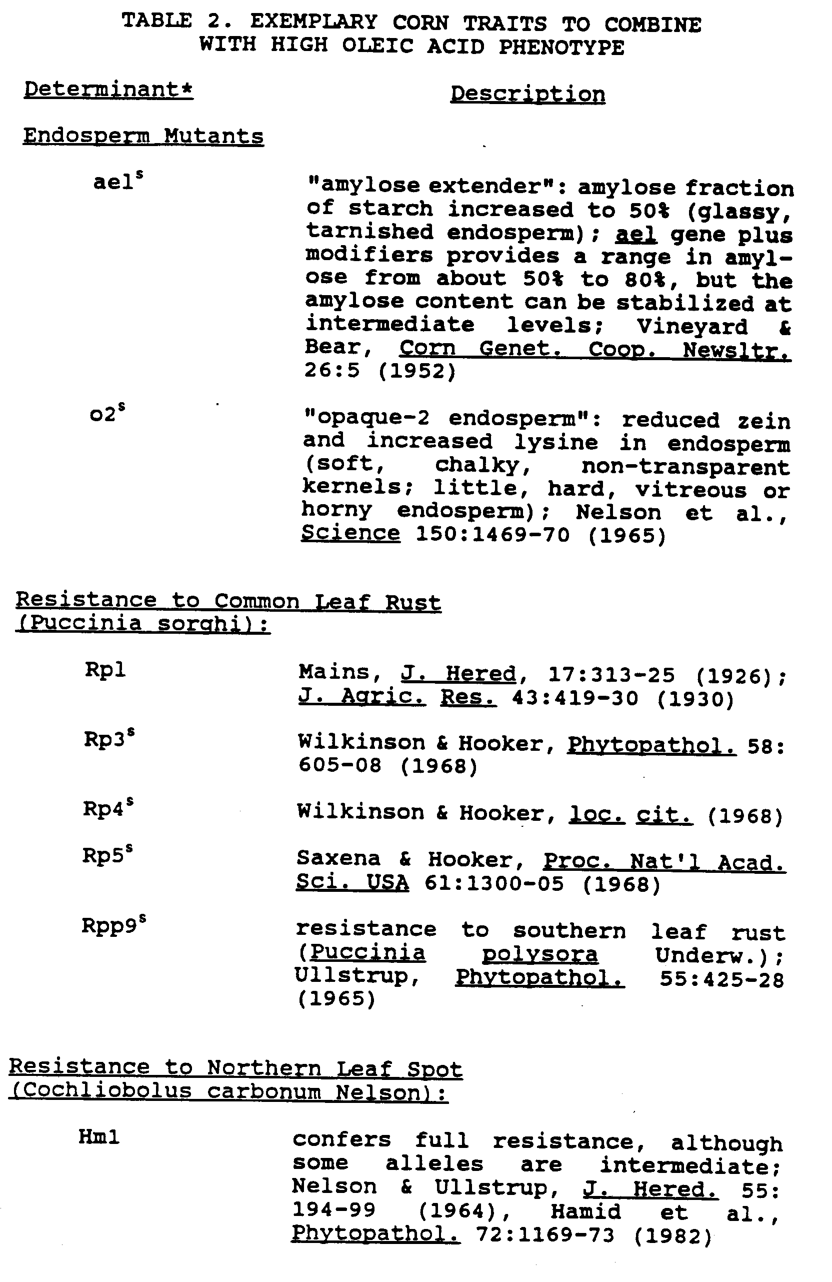

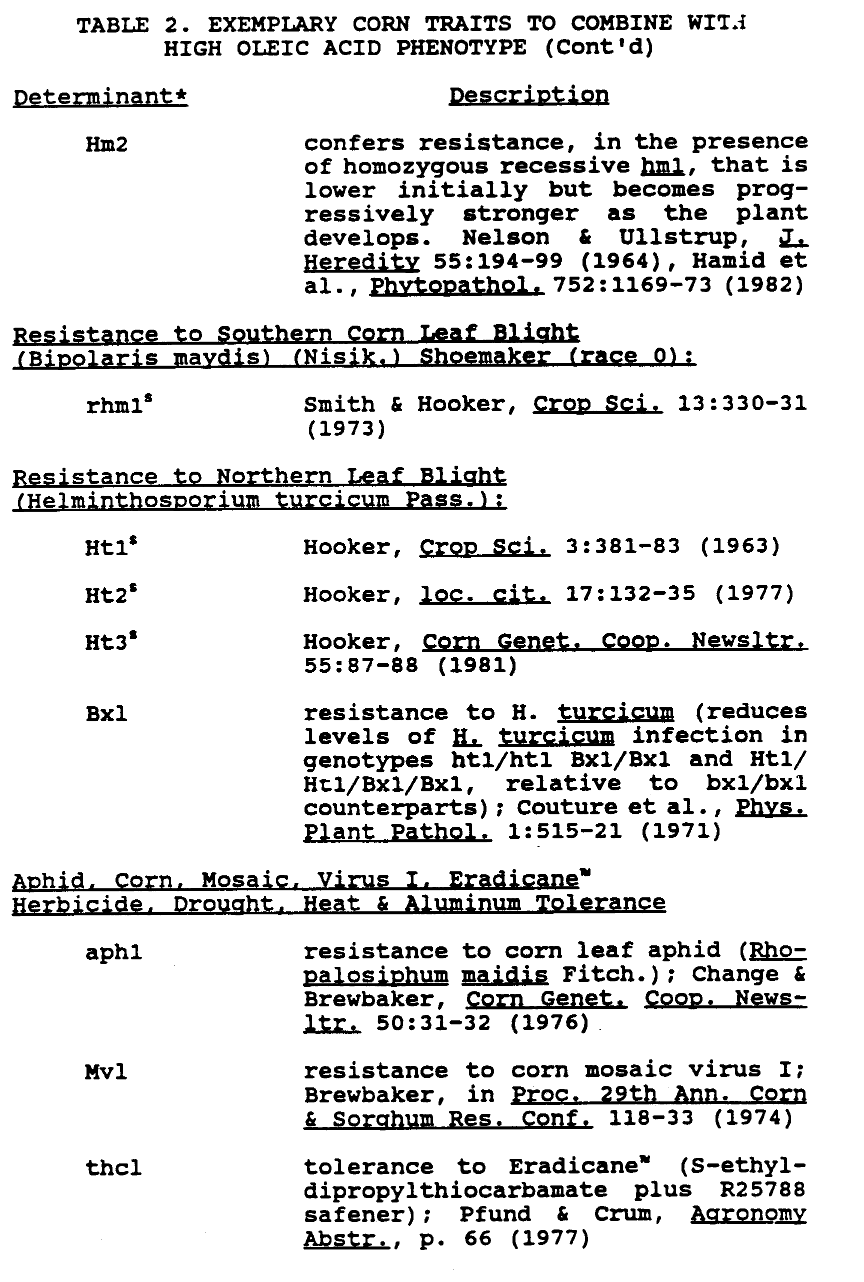

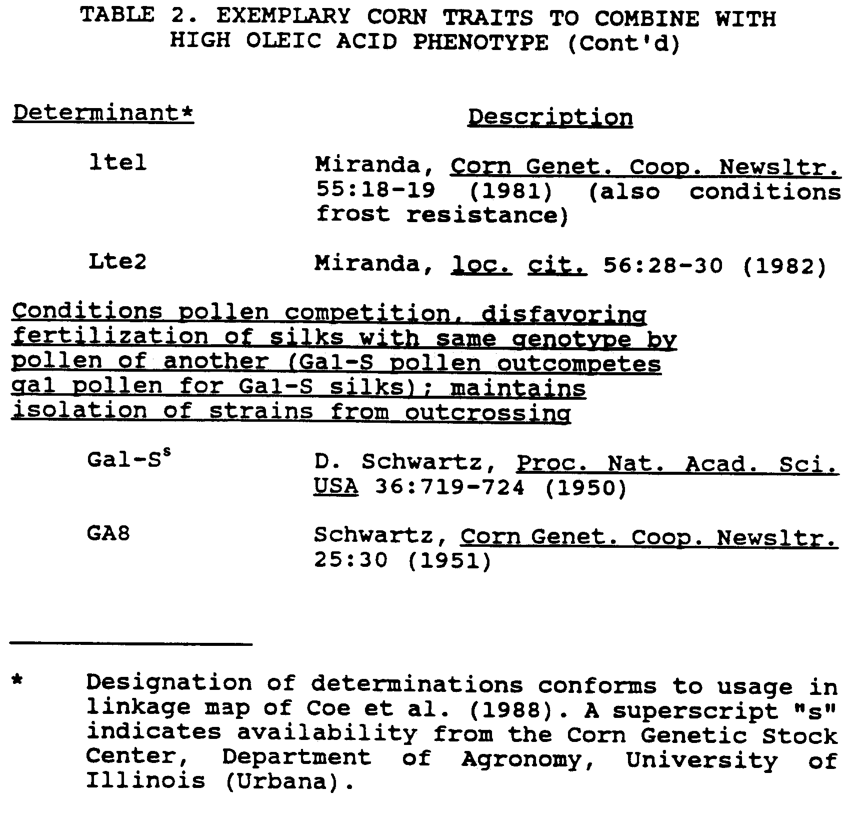

In addition, various approaches to imparting male sterility in corn can be used to produce male-sterile, high oleic acid material within the present invention, which material can be employed in turn to produce hybrids which also display a high oleic acid phenotype according to the present invention. An inbred line possessing such a high oleic acid phenotype can thus be advantageously employed in a backcrossing program as a recurrent parent to cytoplasmic-genetic, male-sterile donors containing both nuclear and cytoplasmic factors imparting male sterility (A lines), to donors containing nuclear but not cytoplasmic factors imparting male sterility (B lines), and to donors containing nuclear and cytoplasmic factors that restore fertility to male-sterile material (R lines), as described, for example, by Coe et al. (1988), at pages 195-98 and pages 206-09, and Poehlman, "Breeding Field Crops" (2d ed.), AVI Publishing Co. (1979), at pages 292-95. Sources for cytoplasmic male-sterility (cms) and fertility restoration (Rf) factors include Holden's Foundation Seeds, Inc., P.O. Box 839, Williamsburg_{,} Iowa 52361 (cms-S and cms-C); Illinois Foundation Seeds, Inc., P.O. Box 722, Champaign, Illinois 61820 (cms-S and cms-C); and Agronomy Department, University of Illinois, Urbana, Illinois (cms-T, cms-S, cms-C and various Rf determinations).

### EXAMPLES

The following examples are provided to further illustrate the present invention and are not intended to limit the invention beyond the limitations set forth in the appended claims.

### EXAMPLE 1

### Determination of Fatty Acid Content

The fatty acid composition of corn seeds developed in the breeding program was determined by GLC in accordance with the procedures described below.

The oil was obtained by using a corn oil extraction protocol having the following steps:
1. A sample corn was removed from the ear and was then catalogued according to row number and pedigree.
2. The sample was crushed with a pestle in a mortar. Ether was added and the sample was crushed further for ten seconds.
3. This solution was then drawn up through non-absorbing cotton into a pipette. The clean solution was then put into a test tube.
4. Three drops of tetramethylammonium hydroxide or sodium methoxide were added to the solution in the test tube and allowed to react for five minutes.
5. After five minutes, distilled water was added to the solution to raise the liquid level in the test tube. Since oil is lighter than water, the oil was easily siphoned off the top layer.
6. The oil drawn off was placed in 2 ml vials. Ether was added to raise the level to three-fourths full.
7. The vial was then capped and thus ready to be processed through the gas liquid chromatograph.

For the analyses of one-half seed samples, individual seeds were cut in half with a razor blade. The halves containing the scutella were then placed in a mortar with a small amount of ether, approximately 1 to 1.5 ml. The pieces were crushed and stirred for approximately 10 seconds with a pestle, and the solution drawn up through non-absorbent cotton and placed in a test tube. A 0.5 ml sample of the ether extractant was then treated in the manner described above. The fatty acid analysis of one half-seed allowed planting of the remaining half-kernel in a breeding nursery and conducting of additional research and development with this genotype.

The GLC analyses were accomplished using a 5890A Hewlett-Packard gas liquid chromatograph equipped with a flame ionization detector and a Hewlett-Packard 3396A integrator. The column used was a Supelco 2330 fused silica capillary column (having a film thickness of 0.2 micron and column dimensions of 15 m. x 0.25 mm.). The operating conditions for the GLC analysis included an injector temperature of 250 degrees Celsius and a detector temperature of 300 degrees Celsius. Column flow was 2.0 ml/min. of helium. Each chromatographic run was temperature-programmed to begin at 170 degrees Celsius and remain at that temperature for 1.0 min. The temperature was then increased to 180 degrees Celsius at a rate of 1 or 2 degrees Celsius/min. After this period of time, the chromatograph was completed and the column prepared for the next run.

### EXAMPLE 2

### Development of High Oleic Acid Corn Lines 85-205-6 and 85-205-21 from Plant Introduction Germplasm

In an exemplary embodiment of the present invention, seed from P.I.'s representing corn material within the above-defined subgroup of suitable starting materials was obtained from the USDA Plant Introduction Station at Iowa State University and, after a grow-out and selfing step, seeds from ears of the selfed P.I.'s were screened for low linoleic acid content via GLC. Prior to 1982, at least two cycles of recurrent selection for low linoleic acid level had occurred. P.I.'s were the progenitors of the recurrent selection population.

Sibbing in 1982 was conducted by mixing pollen of five to eight plants from the first seven rows and pollinating plants in the second eight rows, and vice versa, on several days. Eighty sibbed ears were harvested from this block and analyzed for oil composition, with results as shown in Table 3.

**TABLE 3**

| OLEIC ACID VALUES OF 80 S0 SIBBED EARS HARVESTED IN 1982 | | | | | |
|---|---|---|---|---|---|
| Ear | Oleic Acid | Ear | Oleic Acid | Ear | Oleic Acid |
| -1 | 49.77 | -27 | 47.25 | -53 | 49.32 |
| -2 | 47.20 | -28 | 48.23 | -54 | 50.58 |
| -3 | 44.29 | -29 | 46.11 | -55 | 36.93 |
| -4 | 45.13 | -30 | 47.56 | -56 | 40.81 |
| -5 | 48.58 | -31 | 50.06 | -57 | 44.13 |
| -6 | 46.41 | -32 | 50.03 | -58 | 47.75 |
| -7 | 49.16 | -33 | 48.64 | -59 | 47.21 |
| -8 | 48.93 | -34 | 53.81 | -60 | 31.86 |
| -9 | 43.06 | -35 | 50.02 | -61 | 50.99 |
| -10 | 53.15 | -36 | 49.41 | -62 | 48.67 |
| -11 | 52.67 | -37 | 55.21 | -63 | 52.19 |
| -12 | 52.34 | -38 | 51.66 | -64 | 46.57 |
| -13 | 47.05 | -39 | 48.16 | -65 | 52.37 |
| -14 | 48.37 | -40 ** | 53.07 | -66 | 47.75 |
| -15 | 41.06 | -41 | 47.29 | -67 | 50.91 |
| -16 | 48.97 | -42 | 47.13 | -68 | 50.01 |
| -17 | 51.53 | -43 | 44.42 | -69 | 49.03 |
| -18 | 48.91 | -44 | 45.39 | -70 | 43.08 |
| -19 | 53.16 | -45 | 49.42 | -71 | 55.38 |
| -20 | 50.80 | -46 | 42.73 | -72 | 46.76 |
| -21 | 54.01 | -47 | 45.70 | -73 | 49.86 |
| -22 | 43.87 | -48 | 50.44 | -74 | 50.24 |
| -23 | 45.54 | -49 | 43.85 | -75 | 47.68 |
| -24 | 50.18 | -50 | 43.36 | -76 | 45.38 |
| -25 | 43.94 | -51 | 58.98 | -77 | 46.51 |
| -26 | 24.02 | -52 | 52.01 | -78 | 42.88 |
| | | | | -79 | 52.36 |
| | | | | -80 | 40.73 |

| | | | | | |
|---|---|---|---|---|---|
| ** Seed harvested from this ear was used for planting row 9 in 1983. | | | | | |

Ten S0 ears from the 1982 sibbed ears of 80 were grown in 1983 for selfing in one block and in another block for the next cycle of sibbing. One sibbing block of 15 rows was designated as 82-805. A group of 24 selfed ears was analyzed for fatty acid composition with the results shown in Table 4.

**TABLE 4**

| OLEIC ACID VALUES OF 24 SELFED (S1) EARS HARVESTED IN 1983 | | | |
|---|---|---|---|
| Row No. | Oleic Acid | Row No. | Oleic Acid |
| 83-221-1 | 53.45 | -13 | 61.19 |
| -2 | 65.08 | -14 | 54.25 |
| -3 | 58.65 | -15 | 56.62 |
| -4 | 56.79 | -16 | 48.28 |
| -5 | 56.27 | 83-221-17 | 55.11 |
| -6 | 52.01 | -18 | 57.88 |
| -7 | 47.81 | -19 | 54.67 |
| -8 | 51.00 | -20 | 58.45 |
| 83-221-9 ** | 71.13 | -21 | 56.72 |
| -10 | 60.07 | -22 | 51.05 |
| -11 | 58.42 | -23 | 55.06 |
| -12 | 55.85 | -24 | 57.66 |

| | | | |
|---|---|---|---|
| ** Seed harvested from this row was used for planting row 205 in 1985. | | | |

One ear, 83-221-9, was planted in a four-row nursery block (85-205) and selfed in 1985. The nursery notes recorded the plot as having "good plants," which were five to six feet tall. At harvest, the ears were described as "medium small" and "segregating" (as to color, etc.). In the seed room, five ears were discarded and 32 ears were shelled and individually packaged. One ear was identified as 85-205-6, and a second as 85-205-21. GLC assays on 8-10 kernel bulks showed 85-205-21 had an oleic acid percentage from 73.2% to 75.8%, while 85-205-6 had 69.7% mean oleic acid content.

### EXAMPLE 3

### Development of High Oleic Acid Corn Genotypes From 85-205-21

Seeds from genotype 85-205-21 were grown out and the resulting plants were self-pollinated. Single kernels from an S2 ear (10760109-1) analyzed via GLC resulted in oleic acid contents ranging from 67.7% to 73.1%. Selection 10760109-1 was selfed, and GLC assays of half-kernels from S3 ears resulted in oleic values from 66.1% to 76.1%.

Kernel 4 of S3 ear 10833030-11 was selected for planting in the summer nursery at Breckenridge, Minnesota during 1988. This plant was self-pollinated. Half-seed assays from the selfed ear with the highest oleic values revealed a level of homogeneity, in terms of oleic value for that ear (five kernels assayed: 77.3%, 76.8%, 78.1%, 80.9%, 80.3%). Half-seed assays of other S4 ear seeds obtained from selfing other selected S3 genotypes yielded the highest oleic value (82.1%) overall, with a lowest value on the same ear of 70.8%. No ear provided an oil from bulked seed that was less than 64.1% in oleic value. Further selfing results in lines which are true-breeding for a mean oleic acid content in excess of 65%.

With the high oleic acid corn material of the present invention, corn oil can be produced having a higher percentage of oleic acid than any achieved heretofore, one comparable to commercially-available rapeseed (canola) oil and higher than most sunflower oils. This elevated oleic value is advantageous, both from the standpoint of increased oxidative stability for the oil and the health benefits associated with dietary substitution of monounsaturated for polyunsaturated fatty acids.

### EXAMPLE 4

### Development of Commercially Acceptable, High Oleic Corn Genotypes

In the 1987 summer nursery at Breckenridge, the fatty acid corn material 85-205-6 was grown. The inbred line LH85, in row 8701013923, was used as the female parent in a cross with plant number 4 of 85-205-6 in row 8701013832. Single kernels of LH85 have oleic acid contents of 26.1% to 35.9%. LH85 is a proprietary line available under license from Holden's Foundation Seeds, Inc., and developed from selfing directly out of Pioneer Hi-Bred International's 3978 hybrid. The pollen parent (85-205-6) was a plant grown from seed which showed a 69.7% mean oleic acid content when a bulk of 10 kernels was previously assayed (see Example 2).

In the 1987 fall greenhouse at Breckenridge, the F1 plant from the above-stated cross were grown in row 10710121 and selfed. An assay of approximately five kernels from the third plant showed a mean oleic acid content of 46.7%.

In the 1988 summer nursery, the seed from the 1987 fall greenhouse was planted. Resulting plants were selfed. When harvested, all ears were placed in a bag. Half-kernel GLC assays of the 14th kernel from the seventh ear revealed an oleic acid content of 75.3%. In the 1989 spring greenhouse, the one-half kernel was germinated, transplanted, and labelled as the first plant in row 10821193. This plant was selfed. No assays were performed on seeds from this plant.

In the 1989 summer nursery at Breckenridge, seed of the selfed plant (10821193) was grown in rows 10901914 and 10901915. All plants were selfed. Ears were harvested and placed into a bag labelled 10901914. Assays of five kernel bulks were performed on seeds from ears 1, 2, 3, 4, 5, 6, 7, 8, 9, 10, 11, 12, 13, 14 and 15. One-half kernel assays were then conducted on single kernels from all ears except for ears 8, 11 and 12. With the exception of one seed in ear 14, all oleic acid contents were at least 70.9%. The lower oleic acid content of the seed from ear 13 was most likely accidental out crosses to plants having lower oleic acid genotypes. Results of the GLC assays are shown in Tables 5 and 6.

**TABLE 5**

| OLEIC ACID VALUES BY EAR | | | | |
|---|---|---|---|---|
| Ear | 5 Kernel Bulk | Single Kernels | | |
| | | High | Low | Mean |
| 1 | 76.8 | 81.3 | 72.8 | 76.8 |
| 2 | 76.7 | 76.6 | 75.6 | 76.2 |
| 3 | 75.2 | 79.5 | 74.5 | 77.4 |
| 4 | 75.7 | 77.3 | 74.6 | 75.6 |
| 5 | 77.0 | 79.0 | 70.9 | 76.6 |
| 6 | 75.0 | 79.1 | 76.2 | 77.2 |
| 7 | 76.8 | 80.8 | 74.8 | 78.2 |
| 8 | 74.5 | -- | -- | -- |
| 9 | 75.1 | 77.0 | 72.3 | 75.8 |
| 10 | 76.1 | 78.0 | 75.6 | 76.8 |
| 11 | 73.9 | -- | -- | -- |
| 12 | 73.5 | -- | -- | -- |
| 13 | 69.7 | 74.4 | 60.0 | 71.3 |
| 14 | 77.5 | 81.1 | 78.6 | 79.6 |
| 15 | 77.2 | 77.6 | 73.3 | 76.4 |

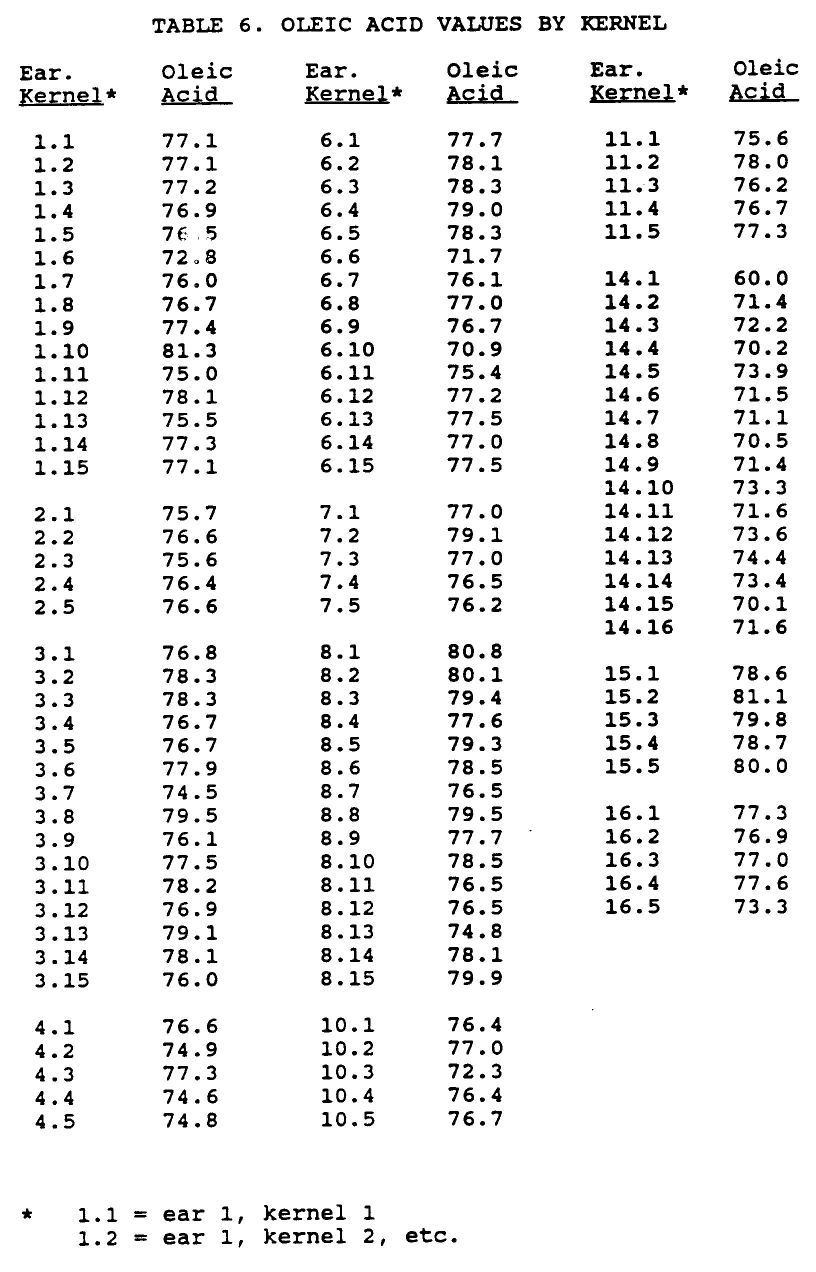

By following the above procedure and using the following elite corn varieties in place of LH85, high oleic corn lines are obtained having mean oleic acid contents greater than 45%.

| | Oleic Acid % |
|---|---|
| McCurdy's 74-41 (MC 74-41) | 31.8 - 34.2 |
| B73 | 22.0 - 39.8 |
| Mo17 | 19.2 - 22.9 |
| M50-1 | 18.1 - 25.6 |
| PA91 | 29.7 - 32.5 |
| Jacques 17 (J17) | 20.7 - 32.0 |
| McCurdy's 731 (MC731) | 23.9 - 26.2 |
| McCurdy's 75-103 (MC75-103) | 25.3 - 27.5 |
| McCurdy's 851 (MC851) | 32.6 - 34.7 |

By following the above procedure and using any commercially acceptable elite corn lines, high oleic corn lines are obtained having a mean oleic acid content greater than 45%.

### REFERENTIAL EXAMPLE 5

### Development of High Oleic Corn Line Using PA91 as the Recurrent Parent

PA91 was crossed with previously identified high oleic line 85-205-21 in the Breckenridge winter greenhouse in 1989. PA91 is an elite, 128-day maturity corn inbred developed at Pennsylvania State University. PA91 was selected from the genetic background of [(Wf9 x Oh40B)⁴] x [(Ind 38-11 x L317) Ind 38-11⁴]. 85-205-21 is a high oleic line developed in Example 2 above. The seed of this cross was grown at Lincoln, Illinois and the first backcross was then made to recurrent parent PA91. Seed of the first backcross was planted in Puerto Rico, where the plants were selfed. The five-kernel bulk GLC assay of BC1 selfed ear 19 had 56.4% oleic (see Table 7). The half-kernel GLC assay of kernel 31 from ear 19 had a 72.4% oleic acid content (see Table 8). Individual kernels identified in Table 8 with a superscript "1" in the first column (row number) are backcrossed to the elite PA91 parent.

**TABLE 7**

| FATTY ACID CONTENT OF OIL FROM FIVE KERNEL BULK OF PEDIGREE PA91²HO) BCl | | | | |
|---|---|---|---|---|
| ROW NO.-EAR | OIL COMPOSITION (%) | | | |
| | PALM 16:0 | STEA 18:0 | OLEI 18:1 | LINO 18:2 |
| 3908698-1 | 8.9 | 2.1 | 47.2 | 41.0 |
| -2 | 9.7 | 1.5 | 44.5 | 44.3 |
| -3 | 12.4 | 2.0 | 47.7 | 37.0 |
| -4 | 12.0 | 1.8 | 40.7 | 45.5 |
| -5 | 10.3 | 2.1 | 35.8 | 51.9 |
| -6 | 10.9 | 1.9 | 39.4 | 47.1 |
| -7 | 11.2 | 1.8 | 53.5 | 32.8 |
| -8 | 11.8 | 2.2 | 34.2 | 51.7 |
| -9 | 11.0 | 1.8 | 46.9 | 39.6 |
| -10 | 13.4 | 2.0 | 34.2 | 49.7 |
| -11 | 8.5 | 1.8 | 36.3 | 52.6 |
| 3908690-12 | 13.1 | 2.0 | 36.4 | 48.5 |
| -13 | 12.9 | 1.7 | 30.3 | 55.1 |
| -14 | 11.1 | 2.5 | 36.5 | 49.0 |
| -15 | 11.2 | 1.5 | 55.4 | 30.8 |
| -16 | 13.1 | 1.7 | 46.5 | 38.7 |
| -17 | 12.1 | 1.9 | 43.4 | 41.8 |
| -18 | 10.7 | 2.1 | 41.4 | 44.8 |
| -19 | 9.1 | 2.1 | 56.4 | 31.1 |
| -20 | 8.3 | 1.9 | 35.6 | 53.3 |

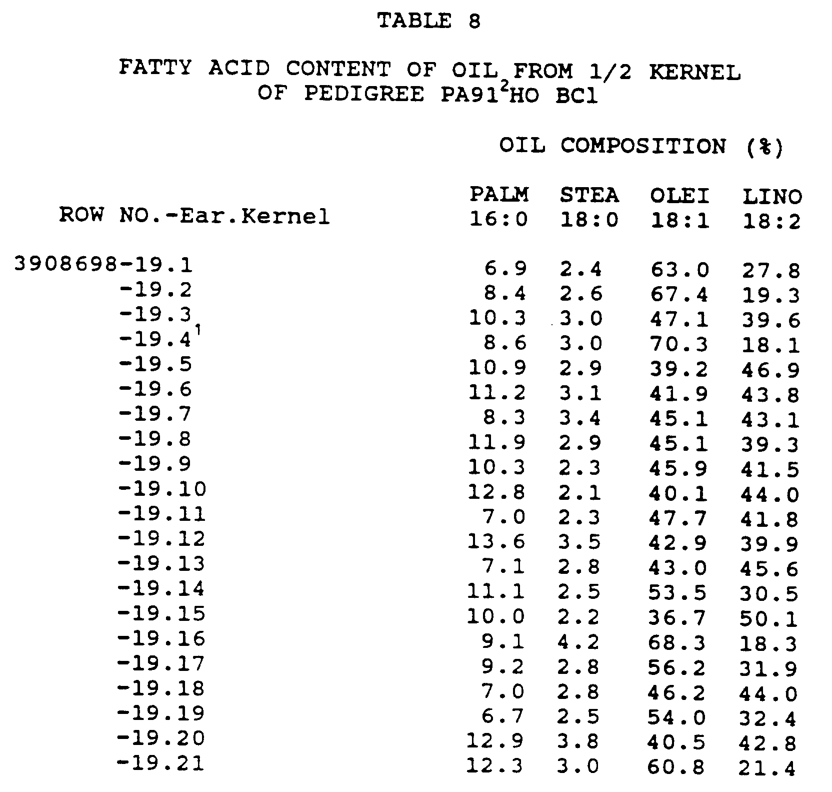

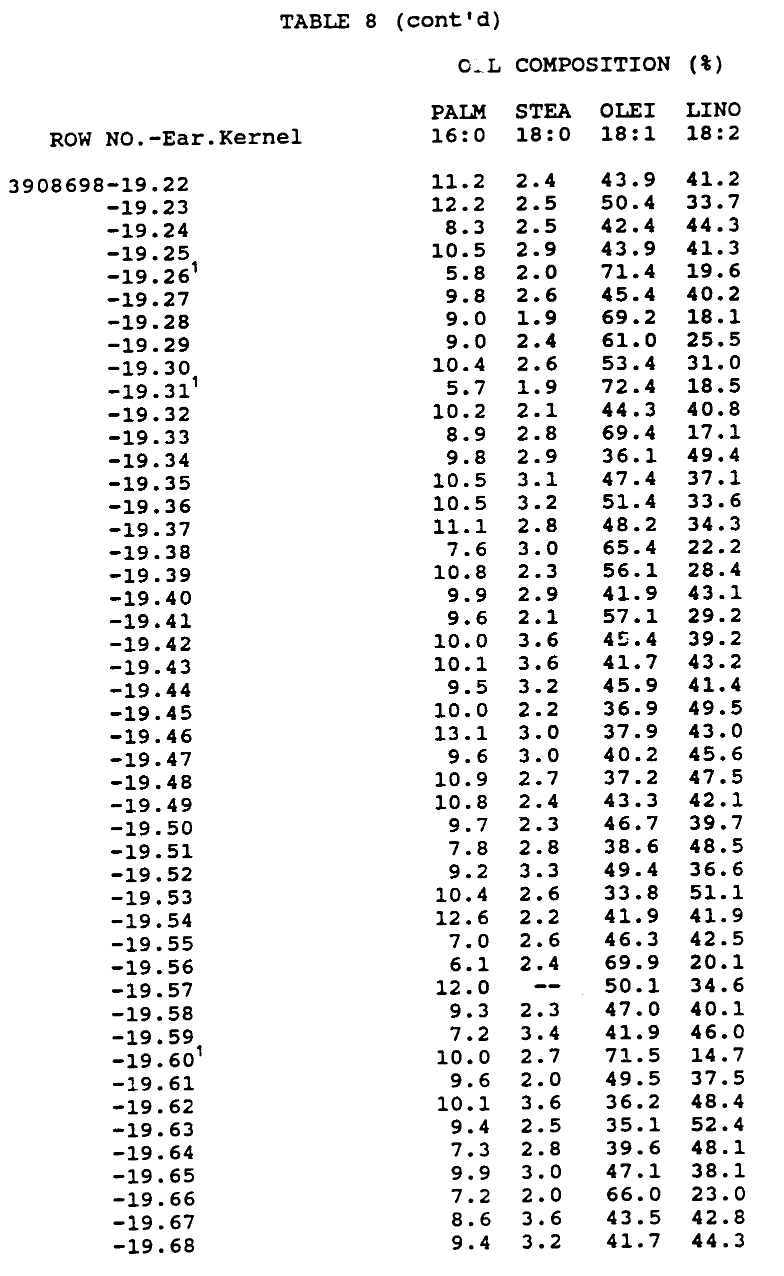

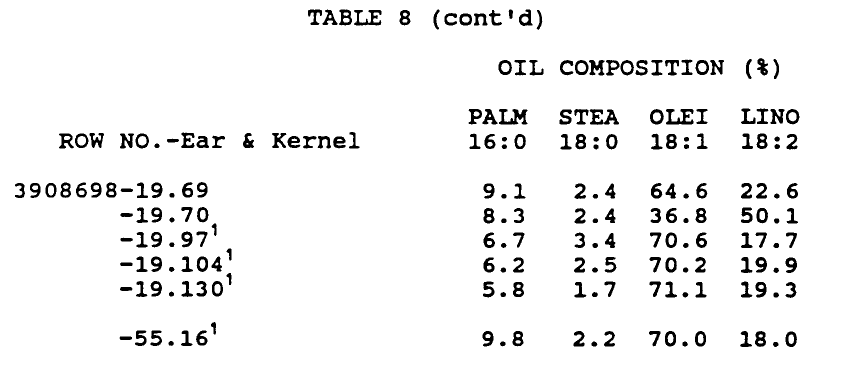

Elite corn inbreds having substantially the same characteristics as PA91, but with the high oleic acid trait from 85-205-21, are produced in two to six backcrosses by backcrossing to the elite parent and then selfing and selecting high oleic acid segregates.

### EXAMPLE 6

### Development of High Oleic Corn Line Using MC731 as the Recurrent Parent

In another embodiment to the present invention, one backcross was made to elite parent MC731 to incorporate a higher percentage of the elite germplasm characteristics of MC731, while retaining the high oleic trait.

High oleic corn line 85-205-21 was crossed with MC731. MC731 is a proprietary line developed by McCurdy's Seed Company, Fremont, Iowa. The background of MC731 is B73²N7A. B73²N7A is an abbreviation of the cross (B73 x N7A)B73. The background of 85-205-21 is described in Example 2.

The MC731 x (85-205-21) cross was backcrossed to MC731. Five kernel bulks of individual S1 ears from this cross were assayed using the GLC methods described previously in Example 1. As shown in Table 9, ear 1 from row 151 has an oleic value of 67.5%. Upon further GLC analysis of individual kernels from ear 1 of row 151 (shown in Table 10), kernel 4 of ear 1 had an oleic percentage of 73.9%, kernel 27 had a percentage of 72.3% and kernel 38 had a percentage of 75.3%. These values are greater than the maximum oleic acid percentage previously reported in corn. Kernels identified in Table 10 with a superscript "1" in the first column (row number) are backcrossed to the MC731 parent. High oleic inbreds having substantially the same characteristics as MC731 are produced in accordance with Referential Example 5. Alternatively, the kernels are further selfed to develop elite lines.

**TABLE 9**

| FATTY ACID CONTENT OF OIL FROM FIVE KERNEL BULK OF PEDIGREE MC731²HO BCl | | | | |
|---|---|---|---|---|
| ROW NO.-Ear | OIL COMPOSITION (%) | | | |
| | PALM 16:0 | STEA 18:0 | OLEI 18:1 | LINO 18:2 |
| 143-1 | 7.8 | 1.6 | 41.5 | 48.1 |
| 143-2 | 8.8 | 1.7 | 54.9 | 36.6 |
| 143-3 | 8.8 | 1.7 | 44.1 | 44.5 |
| 144-1 | 7.3 | 2.5 | 29.6 | 59.6 |
| 144-2 | 9.3 | 1.9 | 46.9 | 40.1 |
| 144-3 | 8.1 | 2.0 | 31.2 | 57.8 |
| 144-4 | 7.2 | 2.5 | 42.8 | 46.5 |
| 144-5 | 9.8 | 1.5 | 36.0 | 51.8 |
| 145-1 | 9.9 | 2.6 | 51.8 | 34.7 |
| 146-1 | 7.4 | 1.9 | 44.2 | 45.6 |
| 147-1 | 6.8 | 2.0 | 46.0 | 45.2 |
| 148-1 | 9.1 | 2.5 | 67.2 | 20.4 |
| 149-1 | 8.2 | 1.7 | 51.1 | 38.0 |
| 150-1 | 9.0 | 2.4 | 35.4 | 52.3 |
| 150-2 | 7.2 | 1.7 | 45.3 | 45.7 |
| 151-1 | 6.8 | 2.0 | 67.5 | 23.7 |
| 152-1 | 6.9 | 2.4 | 61.8 | 28.4 |
| 153-1 | 9.3 | 2.3 | 38.6 | 48.8 |

**TABLE 10**

| FATTY ACID CONTENT OF OIL FROM 1/2 KERNEL OF PEDIGREE MC731²HO BCl | | | | |
|---|---|---|---|---|
| ROW NO.-Ear.Kernel | OIL COMPOSITION (%) | | | |
| | PALM 16:0 | STEA 18:0 | OLEI 18:1 | LINO 18:2 |
| 151-1.1 | 8.5 | 1.9 | 35.0 | 53.0 |
| 1.2 | 7.8 | 2.6 | 37.8 | 50.2 |
| 1.3 | 8.2 | 2.1 | 51.9 | 36.3 |
| 1.4¹ | 7.5 | 2.5 | 73.9 | 14.8 |
| 1.5 | 7.7 | 2.0 | 49.5 | 39.3 |
| 1.6 | 9.4 | 2.2 | 49.8 | 37.4 |
| 1.7 | 9.0 | 2.3 | 42.6 | 43.9 |
| 1.8 | 8.2 | 2.5 | 45.9 | 41.9 |
| 1.9 | 6.5 | 2.1 | 54.7 | 35.5 |
| 1.10 | 8.8 | 2.1 | 49.8 | 39.3 |
| 1.11 | 7.3 | 2.2 | 69.5 | 19.4 |
| 1.12 | 9.9 | 1.6 | 36.4 | 50.6 |
| 1.13 | 8.7 | 2.1 | 46.9 | 40.7 |
| 1.14 | 10.2 | 2.8 | 48.9 | 38.2 |
| 1.15 | 8.1 | 1.8 | 66.4 | 22.2 |
| 1.16 | 9.4 | 2.3 | 47.0 | 39.3 |
| 1.17 | 7.0 | 2.5 | 55.4 | 32.2 |
| 1.18 | 7.9 | 2.5 | 43.5 | 44.1 |
| 1.19 | 7.4 | 1.9 | 50.9 | 38.4 |
| 1.20 | 6.3 | 2.0 | 54.7 | 36.1 |
| 1.21 | 5.4 | 2.1 | 41.2 | 50.1 |
| 1.22 | 10.0 | 2.4 | 44.5 | 41.7 |
| 1.23 | 8.1 | 2.0 | 63.4 | 24.9 |
| 1.24 | 5.7 | 1.6 | 47.6 | 43.4 |
| 1.25 | 6.5 | 2.2 | 50.4 | 39.6 |
| 1.26 | 9.9 | 2.7 | 50.9 | 35.1 |
| 1.27¹ | 6.8 | 2.3 | 72.3 | 17.0 |
| 1.28 | 5.9 | 2.1 | 47.9 | 42.5 |
| 1.29 | 9.6 | 2.3 | 40.5 | 46.1 |
| 1.30 | 7.1 | 2.2 | 36.7 | 52.4 |
| 1.31 | 7.7 | 2.1 | 50.9 | 37.5 |
| 1.32 | 10.1 | 1.8 | 27.4 | 59.1 |
| 1.33 | 8.0 | 2.8 | 45.7 | 41.3 |
| 1.34 | 8.5 | 2.1 | 37.1 | 50.7 |
| 1.35¹ | 6.7 | 2.3 | 70.5 | 19.3 |
| 1.36 | 6.5 | 2.6 | 42.0 | 47.4 |
| 1.37¹ | 7.5 | 2.2 | 72.3 | 16.6 |
| 1.38¹ | 6.2 | 2.5 | 75.3 | 14.2 |
| 1.39 | 7.5 | 2.9 | 34.9 | 53.1 |
| 148-1.7¹ | 8.9 | 2.4 | 70.9 | 16.5 |

By following the procedures described in Example 6 and using any commercially acceptable elite corn line, high oleic acid corn lines may be obtained having a mean oleic acid content greater than 75%.

### EXAMPLE 7

### Development of High Oleic Acid Waxy Corn Lines

Simultaneously with the backcrossing procedures of Examples 5-11, whereby elite endosperm lines are being converted to high oleic acid genotypes, another backcross procedure is employed to convert MC74-41, PA91, J17, MC731, MC75-103 and MC851 to waxy endosperm genotypes. The latter backcross procedure comprises the following steps: (1) crossing the elite (recurrent) normal endosperm parent to the waxy (donor) parent, yielding F1 seed; (2) backcrossing the F1 to the recurrent parent; (3) growth and self-pollination of the BC1 progeny; and (4) after harvest of the mature seed, identification of waxy kernels which then become the seed for the next backcross to the normal, recurrent parent (steps two and three above).

Some of the high oleic acid seed from Examples 5-11 are used in backcrossing progeny in which the waxy parents, converted from normal to waxy kernel endosperms by the above procedure, become the recurrent parents. For example, waxy PA91 is used as the recurrent parent in a backcrossing procedure with the high oleic line produced in Example 5. Since only waxy, high oleic seeds are the desired product, a four-fold population size is necessary since the waxy allele is recessive. In other words, only one-fourth of the high oleic recoveries will have waxy endosperms.

Normally three to five backcrosses are sufficient to recover the phenotype of the recurrent parent in a plant that produces waxy kernels.

## Claims (Claims for the following Contracting State(s): AT, BE, CH, LI, DE, DK, FR, GB, GR, IT, LU, NL, SE)

1. A corn oil having a mean oleic acid content of at least 75% relative to the total oil which has been extracted from a substantially homogeneous assemblage of corn seeds having a mean oleic acid content of at least 75% by weight relative to the total fatty acid content of said seeds.

2. An oil according to claim 1, wherein said oleic acid content is greater than about 82% by weight.

3. An oil according to any preceding claim, wherein said corn seeds are obtained from a corn plant which is a hybrid.

4. An oil according to claim 3 wherein said hybrid displays a waxy phenotype.

5. An oil according to either of claims 3 and 4 wherein said hybrid displays an amylose extender genotype.

6. An oil according to either of claims 3 and 4 wherein said hybrid displays a Gal-S (super gametophyte factor) genotype.

7. An oil according to any preceding claim, extracted from seeds wherein at least some of said seeds, if germinated, would grow into plants that also produce seeds having a mean oleic acid content of at least 75% by weight relative to the total fatty acid content of said seeds.

8. An oil according to claim 1 extracted from Zea mays L. seed product consisting of a substantially homogeneous assemblage of corn seeds which has a mean oleic acid content of at least 75% by weight relative to the total fatty acid content of said seeds.

## Claims (Claims for the following Contracting State(s): ES)

1. A process for the preparation of a corn oil having a mean oleic acid content of at least 75% relative to the total oil which comprises extracting said oil from a substantially homogeneous assemblage of corn seeds having a mean oleic acid content of at least 75% by weight relative to the total fatty acid content of said seeds.

2. A process according to claim 1, wherein said oleic acid content is greater than about 82% by weight.

3. A process according to any preceding claim, wherein said corn seeds are obtained from a corn plant which is a hybrid.

4. A process according to claim 3 wherein said hybrid displays a waxy phenotype.

5. A process according to either of claims 3 and 4 wherein said hybrid displays an amylose extender genotype.

6. A process according to either of claims 3 and 4 wherein said hybrid displays a Gal-S (super gametophyte factor) genotype.

7. A process according to any preceding claim wherein at least some of said seeds, if germinated, would grow into plants that also produce seeds having a mean oleic acid content of at least 75% by weight relative to the total fatty acid content of said seeds.

8. A process according to claim 1 wherein said oil is extracted from Zea mays L. seed product consisting of a substantially homogeneous assemblage of corn seeds which has a mean oleic acid content of at least 75% by weight relative to the total fatty acid content of said seeds.

## Patentansprüche (Patentansprüche für folgende(n) Vertragsstaat(en): AT, BE, CH, LI, DE, DK, FR, GB, GR, IT, LU, NL, SE)

1. Maisöl mit einem mittleren Ölsäuregehalt von wenigstens 75 %, bezogen auf das Gesamtöl, welches aus einer im wesentlichen homogenen Ansammlung von Maiskörnern mit einem mittleren Ölsäuregehalt von wenigstens 75 Gew. % in bezug auf den Gesamtfettsäuregehalt dieser Körner extrahiert wurde.

2. Öl nach Anspruch 1, bei dem der Ölsäuregehalt größer als etwa 82 Gew.% ist.

3. Öl nach einem der vorausgehenden Ansprüche, bei dem die Maiskörner von einer Maispflanze erhalten werden, die ein Hybrid ist.

4. Öl nach Anspruch 3, bei dem das Hybrid einen wachsartigen Phenotyp darstellt.

5. Öl nach einem der Ansprüche 3 und 4, bei dem das Hybrid einen Amylosestreckmittelgenotyp darstellt.

6. Öl nach einem der Ansprüche 3 und 4, bei dem das Hybrid einen Gal-S (Supergametophytfaktor)-Genotyp darstellt.

7. Öl nach einem der vorausgehenden Ansprüche, extrahiert aus Körnern, wobei wenigstens einige dieser Körner, wenn gekeimt, zu Pflanzen wachsen würden, die auch Körner mit einem mittleren Ölsäuregehalt von wenigstens 75 Gew. %, bezogen auf den Gesamtfettsäuregehalt dieser Körner, erzeugen.

8. Öl nach Anspruch 1, extrahiert aus *Zea mays L*.-Samenprodukt, das aus einer im wesentlichen homogenen Ansammlung von Maiskörnern besteht, welche einen mittleren Ölsäuregehalt von wenigstens 75 Gew. %, bezogen auf den Gesamtfettsäuregehalt dieser Körner, hat.

## Patentansprüche (Patentansprüche für folgende(n) Vertragsstaat(en): ES)

1. Verfahren zur Herstelung eines Maisöls mit einem mittleren Ölsäuregehalt von wenigstens 75 %, bezogen auf das Gesamtöl, indem man das Öl aus einer im wesentlichen homogenen Ansammlung von Maiskörnern mit einem mittleren Ölsäuregehalt von wenigstens 75 Gew. % in bezug auf den Gesamtfettsäuregehalt dieser Körner extrahiert.

2. Verfahren nach Anspruch 1, bei dem der Ölsäuregehalt größer als etwa 82 Gew. % ist.

3. Verfahren nach einem der vorausgehenden Ansprüche, bei dem die Maiskörner von einer Maispflanze erhalten werden, die ein Hybrid ist.

4. Verfahren nach Anspruch 3, bei dem das Hybrid einen wachsartigen Phenotyp darstellt.

5. Verfahren nach einem der Ansprüche 3 und 4, bei dem das Hybrid einen Amylosestreckmittelgenotyp darstellt.

6. Verfahren nach einem der Ansprüche 3 und 4, bei dem das Hybrid einen Gal-S (Supergametophytfaktor)-Genotyp darstellt.

7. Verfahren nach einem der vorausgehenden Ansprüche, bei dem wenigstens einige dieser Körner, wenn gekeimt, zu Pflanzen wachsen würden, die auch Körner mit einem mittleren Ölsäuregehalt von wenigstens 75 Gew. %, bezogen auf den Gesamtfettsäuregehalt dieser Körner, erzeugen.

8. Verfahren nach Anspruch 1, bei dem das Öl aus *Zea mays L*.-Samenprodukt extrahiert wird, das aus einer im wesentlichen homogenen Ansammlung von Maiskörnern besteht, welche einen mittleren Ölsäuregehalt von wenigstens 75 Gew. %, bezogen auf den Gesamtfettsäuregehalt dieser Körner, hat.

## Revendications (Revendications pour l'(les) Etat(s) contractant(s) suivant(s): AT, BE, CH, LI, DE, DK, FR, GB, GR, IT, LU, NL, SE)

1. Huile de maïs ayant une teneur moyenne en acide oléique d'au moins 75 %, par rapport à l'huile totale, qui a été obtenue par extraction à partir d'un ensemble essentiellement homogène de graines de maïs ayant une teneur moyenne en acide oléique d'au moins 75 % en poids, par rapport à la teneur totale en acides gras desdites graines.

2. Huile suivant la revendication 1, dans laquelle la teneur en acide oléique est supérieure à environ 82 % en poids.

3. Huile suivant l'une quelconque des revendications précédentes, dans laquelle les graines de maïs sont obtenues à partir d'un plant de maïs qui est un hybride.

4. Huile suivant la revendication 3, dans laquelle l'hybride présente un phénotype cireux.

5. Huile suivant l'une des revendications 3 et 4, dans laquelle l'hybride présente un génotype élément d'extension amylose.

6. Huile suivant l'une des revendications 3 et 4, dans laquelle l'hybride présente un génotype Gal-S (facteur supergamétophyte).

7. Huile suivant l'une quelconque des revendications précédentes, obtenue par extraction à partir de graines dans lesquelles au moins certaines desdites graines, si elles germaient, se développeraient en donnant des plants produisant également des graines ayant une teneur moyenne en acide oléique d'au moins 75 % en poids, par rapport à la teneur totale en acides gras desdites graines.

8. Huile suivant la revendication 1, obtenue par extraction à partir d'un produit consistant en graines de Zea mays L. consistant en un ensemble essentiellement homogène de graines de maïs qui a une teneur moyenne en acide oléique d'au moins 75 % en poids, par rapport à la teneur totale en acides gras desdites graines.

## Revendications (Revendications pour l'(les) Etat(s) contractant(s) suivant(s): ES)

1. Procédé pour la préparation d'une huile de maïs ayant une teneur moyenne en acide oléique d'au moins 75 %, par rapport à l'huile totale, qui comprend l'extraction de ladite huile d'un ensemble essentiellement homogène de graines de maïs ayant une teneur moyenne en acide oléique d'au moins 75 % en poids, par rapport à la teneur totale en acides gras desdites graines.

2. Procédé suivant la revendication 1, dans lequel la teneur en acide oléique est supérieure à environ 82 % en poids.

3. Procédé suivant l'une quelconque des revendications précédentes, dans lequel les graines de mais sont obtenues à partir d'un plant de mais qui est un hybride.

4. Procédé suivant la revendication 3, dans lequel l'hybride présente un phénotype cireux.

5. Procédé suivant l'une des revendications 3 et 4, dans lequel l'hybride présente un génotype élément d'extension amylose.

6. Procédé suivant l'une des revendications 3 et 4, dans lequel l'hybride présente un génotype Gal-S (facteur supergamétophyte).

7. Procédé suivant l'une quelconque des revendications précédentes, dans lequel au moins certaines des graines, si elles germaient, se développeraient en donnant des plants produisant également des graines ayant une teneur moyenne en acide oléique d'au moins 75 % en poids, par rapport à la teneur totale en acides gras desdites graines.

8. Procédé suivant la revendication 1, dans lequel l'huile est extraite d'un produit consistant en graines de Zea mays L. consistant en un ensemble essentiellement homogène de graines de maïs ayant une teneur moyenne en acide oléique d'au moins 75 % en poids, par rapport à la teneur totale en acides gras desdites graines.
